# FASCICULE DE BREVET EUROPEEN

(11) **EP 0 707 486 B1**
(45) Date de publication et mention de la délivrance du brevet: **08.08.2001**
(21) Numéro de dépôt: 95920128.6
(22) Date de dépôt: 09.05.1995
(51) Int. Cl.: A61K 35/56, A61K 7/48

(54) **PRODUIT DE REGENERATION ET DE CICATRISATION CUTANEE, SON PROCEDE DE FABRICATION, SES APPLICATIONS**
MITTEL ZUR REGENERIERUNG UND WUNDHEILUNG DER HAUT, VERFAHREN ZU DESSEN HERSTELLUNG UND VERWENDUNG
CUTANEOUS REJUVENATING AND HEALING PRODUCT, METHOD FOR ITS MANUFACTURE AND USES THEREOF

(30) Priorité: 09.05.1994 FR 9405664
(43) Date de publication de la demande: 24.04.1996
(73) Titulaire: FORTUNE BASE MANAGEMENT, LTD., Central Hong Kong (HK)
(72) Inventeur: Camprasse, Georges, 77480 Villenauxe-la-Petite (FR); Camprasse, Serge, 77500 Chelles (FR)
(74) Mandataire: Thinat, Michel
(86) Numéro de dépôt international: FR9500606
(87) Numéro de publication internationale: WO9530426

(56) Documents cités:
- DE-A- 3 437 184
- FR-A- 1 332 198
- US-A- 4 393 045
- PATENT ABSTRACTS OF JAPAN vol. 12, no. 279 (C-517) (3126) 1 Août 1988 & JP,A,63 057 507 (MIKIMOTO SEIYAKU K.K.) 12 Mars 1988

## Description

La présente invention concerne un produit fabriqué à partir des phases minérale et/ou organique issues de Pinctada Maxima, Margaritifera ou de tout autre mollusque aquatiques bivalves, gastropodes ou céphalopodes, destiné à accélérer le processus de régénération cutanée et la cicatrisation des plaies.

On sait que lors des pertes de substance, par plaies, interventions chirurgicales, ou maladies dégénératives, brûlures ou escares, le processus de cicatrisation se manifeste de deux façons différentes :
a) cicatrisation de première intention, par rapprochement et suture des berges de la plaie, protégeant les plans profonds.
b) cicatrisation de deuxième intention, par bourgeonnement conjonctif partant des plans profonds comblant la perte de substance, suivie d'une cicatrisation des plans superficiels. Ce second processus étant nécessairement beaucoup plus long que le précédent.

Pour tenter d'accélérer ces processus, on utilise plusieurs types de substances, dans le but de protéger la plaie, d'en éviter ou de combattre l'infection. Ce sont le plus souvent des crèmes ou des substances à base d'antiseptiques, d'antibiotiques, de vitamines A, et de plantes. Malgré ces précautions et ces traitements les processus de cicatrisation sont quelques fois pris en défaut pour des raisons diverses, intrinsèques ou extrinsèques.

Cependant, quel que soit le type de cicatrisation ou les traitements mis en oeuvre, la régénération tissulaire totale demande un délai variant de 45 à 60 jours.

Le produit selon l'invention est obtenu d'une part directement à partir d'un broyat de cellules du manteau du mollusque, lyophilisées, et d'autre part, d'un broyat du test nacré du mollusque dont on utilise soit la fraction organique, soit la fraction minérale, soit les deux.

Le produit selon l'invention selon un premier mode de fabrication préférentielle s'obtient de la façon suivante : après concassage et broyage du test nacré du mollusque le résidu ainsi obtenu est lavé et nettoyé dans un bac à ultra-sons, puis subit un traitement liquide enzymatique, soit par l'anhydrase carbonique, soit la trypsine ou tout autre enzyme, suivi d'un traitement acide pour désactiver l'enzyme.

Le résidu est lavé à l'eau déminéralisée, soumis à un traitement d'évaporation à 50° pendant 48 heures, puis allongé avec de l'eau déminéralisée et centrifugé. Selon un second mode de fabrication, un dilacérat du manteau du mollusque est soumis à un traitement thermique sous vide pendant 4 heures. La gelée ainsi obtenue est filtrée sur membrane, concentrée par centrifugation et lyophilisée.

Le produit selon l'invention est obtenu en mélangeant les 2 phases afin de réaliser un gel, une crème ou une solution.

Le produit selon l'invention est mélangé à des excipients dont les propriétés chimiques en potentialisent les effets et favorisent la pénétration. Ces excipients peuvent être, sans que la liste en soit limitative : menthol, camphre, benjoin, acide citrique, oxybenzoate de méthyl, alcool cétylique, alcool stéarylique, vaseline officinale, glycérine, eau purifiée, vitamine A, extraits d'algues, hydroxyde de calcium, extrait de coprah.

Le produit selon l'invention a pour but, compte tenu de ses propriétés eutrophiques, cicatrisantes, d'accélérer de manière notable le processus de cicatrisation par une stimulation de l'activité cytoplasmique des fibroblastes induisant une prolifération de ceux-ci dans un délai de quelques heures.

Des expérimentations "in vivo" ont montré que le produit selon l'invention provoque une accélération et une augmentation de volume du bourgeon de cicatrisation ainsi qu'une coaptation très rapide des berges de la plaie.

Les exemples suivants illustrent les applications du produit selon l'invention.

Deux pertes de substances cutanées de diamètre de 10mm intéressant l'épiderme et le derme sont réalisées sur l'animal. L'une est enduite du produit selon l'invention stérilisé, l'autre recouverte d'un pansement occlusif. On observe dans un délai de 48 heures la formation d'un bourgeon de cicatrisation, avec prolifération cellulaire et diminution des deux tiers de la surface cruantée. La cicatrisation totale intervient au bout de 6 jours alors que la perte de substance témoin cicatrise au bout de 17 jours.

Le produit selon l'invention, stérilisé est appliqué sur un mal perforant plantaire évoluant depuis 15 ans. On sait que le mal perforant plantaire est une affection cutanéo-musculaire fréquente chez le diabétique, se caractérisant par une perforation de la voute plantaire au niveau des points d'appui, le talon et les métatarses. Cette perte de substance se présente sous la forme d'une lésion de diamètre et de profondeur variables, dont les bords sont atones par disparition complète de la vascularisation, quelque fois infectée, n'évoluant jamais vers la guérison spontanée et rebelle à tout traitement. Dans l'exemple cité plus haut, on constate au bout d'une semaine, une diminution spectaculaire du diamètre et de la profondeur de la lésion ainsi qu'une nette revascularisation de ses bords. Le bourgeon de cicatrisation comblant la lésion au bout de 15 jours, la guérison complète est obtenue par régénération de la couche cornée dans un délai de 3 semaines.

Un troisième exemple concerne l'utilisation du produit selon l'invention dans son application cosmétologique : après rubéfaction des régions jugales droite et gauche seule l'une des régions est enduite du produit selon l'invention, l'autre servant de témoin. On note dès l'application du produit selon l'invention, une sédation des phénomènes douloureux à types de brûlures, dans les 48 heures une régénération partielle de la couche superficielle de l'épiderme, avec disparition totale du phénomène d'hyperhémie et au bout de 6 jours l'aspect lisse et normal de l'épiderme témoigne d'une régénération tissulaire totale de la couche superficielle.

Un quatrième exemple concerne l'utilisation du produit selon l'invention sur une brûlure de deuxième degré : le produit selon l'invention sous forme de crème, est appliqué sur toute la surface de la zone brûlée. On note dès l'application du produit selon l'invention, la disparition totale de la sensation de brûlure et l'observation clinique montre une diminution notable de la surface cruantée au bout de 48 heures, ainsi que l'apparition d'un début de repigmentation de l'épiderme.

Toutes ces expérimentations ont mis en évidence l'action de stimulation du processus régulé de prolifération cellulaire par le produit selon l'invention ainsi que ses propriétés eutrophiques, antalgiques, cicatrisantes, et antiphlogistiques.

Il appartiendra à l'homme de métier chaque fois qu'il désirera obtenir une action eutrophique, régénératrice, antalgique, cicatrisante et antiphlogistique, de mettre en oeuvre un gel, une crème, une solution, dont le principe actif serait soit le complexe organo-minéral du test nacré de mollusques bivalves tels que Pinctada Maxima ou autres, soit un lyophilisat des cellules du manteau, soit de la fraction organique ou minérale associés à un ou plusieurs des excipients ou substances cités plus haut.

## Revendications

1. Produit de régénération et de cicatrisation cutanée, caractérisé en ce qu'il est obtenu à partir de lyophilisat des cellules du manteau et de broyat de la nacre de mollusques bivalves, subissant un traitement aux ultra sons, suivi d'un traitement enzymatique par l'anhydrase carbonique ou la trypsine, précédant un traitement thermique sous vide.

2. Produit selon la revendication 1, caractérisé en ce qu'il est issu de cellules du manteau et de la nacre de Pinctada Maxima, Margaritifera, ou de tout autre mollusque bivalve gastéropode ou céphalopode.

3. Produit selon la revendication 1 ou 2, caractérisé en ce qu'il subit un traitement mécanique de broyage et par ultra sons.

4. Produit selon l'une quelconque des revendications précédentes, caractérisé en ce qu'il subit un traitement chimique acide de désactivation enzymatique.

5. Produit selon l'une quelconque des revendications précédentes, caractérisé en ce qu'il subit un traitement d'évaporation à 50° pendant 48 heures.

6. Produit selon l'une quelconque des revendications précédentes, caractérisé en ce qu'il est filtré sur membrane, concentré,par centrifugation et lyophilisé.

7. Produit selon l'une quelconque des revendications précédentes, caractérisé en ce qu'il est obtenu en mélangeant les deux phases afin de réaliser un gel, une crème ou une solution.

8. Produit selon l'une quelconque des revendications précédentes, caractérisé en ce qu'il peut être mélangé à des excipients tels que le menthol, le camphre, le benjoin, l'acide citrique, l'oxybenzoate de méthyle, l'alcool stéarylique, la vaseline officinale, la glycérine, l'eau purifiée, la vitamine A, l'extrait d'algues, l'hydroxyde de calcium, l'extrait de coprah ou tout autre vecteur susceptible d'en potentialiser les effets ou d'en favoriser la pénétration.

9. Utilisation du produit selon l'une quelconque des revendications précédentes dans la fabrication d'une composition dermatologique.

10. Utilisation du produit selon l'une quelconque des revendications précédentes pour la fabrication d'un produit cosmétique.

## Patentansprüche

1. Produkt zur Hautregeneration und -vernarbung, dadurch gekennzeichnet, daß es aus Lyophilisat der Mantelzellen und des Schrots des Perlmutts von zweischaligen Muscheltieren gewonnen wird, das eine Behandlung durch Ultraschall, gefolgt von einer enzymatischen Behandlung durch kohlensaure Anhydrase oder Tipsin vor einer thermischen Vakuumbehandlung erfährt.

2. Produkt gemäß Anspruch 1, dadurch gekennzeichnet, daß es aus Mantelzellen und dem Perlmutt von Finctada Maxima, Margaritifera oder jedem anderen zweischaligen Bauchfüßler- oder Kopffüßler-Muscheltier stammt.

3. Produkt gemäß Anspruch 1 oder 2, dadurch gekennzeichnet, daß es eine mechanische Behandlung durch Zermahlen und durch Ultraschall erfährt.

4. Produkt gemäß einem der vorgenannten Ansprüche, dadurch gekennzeichnet, daß es eine chemische saure Behandlung zur enzymatischen Deaktivierung erfährt.

5. Produkt gemäß einem der vorgenannten Ansprüche, dadurch gekennzeichnet, daß es während 48 Stunden eine Verdunstungsbehandlung bei 50° erfährt.

6. Produkt gemäß einem der vorgenannten Ansprüche, dadurch gekennzeichnet, daß es auf einer Membran gefiltert, durch Zentrifugieren konzentriert und lyophilisiert wird.

7. Produkt gemäß einem der vorgenannten Ansprüche, dadurch gekennzeichnet, daß es durch Vermischen der zwei Phasen gewonnen wird, um ein Gel, eine Creme oder eine Lösung zu realisieren.

8. Produkt gemäß einem der vorgenannten Ansprüche, dadurch gekennzeichnet, daß es mit Grundmassen gemischt werden kann, wie zum Beispiel Menthol, Kampfer, Benzoe, Zitronensäure, Methyl-Oxybenzoat, Stearylalkohol, offizineller Vaseline, Glycerin, reinem Wasser, Vitamin A, Algenextrakt, Kalziumhydroxyd, Kopraextrakt oder jedem anderen Vektor, der geeignet ist, die Wirkungen zu potentialisieren oder das Eindringen zu begünstigen.

9. Einsatz des Produkts gemäß einem der vorgenannten Ansprüche bei der Herstellung einer dermatologischen Zusammensetzung.

10. Einsatz des Produktes gemäß einem der vorgenannten Ansprüche zur Herstellung eines kosmetischen Produkts.

## Claims

1. Cutaneous rejuvenating and healing product, characterized in that it is obtained from a lyophilisate of cells of the mantle and a crushing of the mother of pearl of bivalvular molluscs, submitted to an ultrasonic treatment followed by an enzymatic treatment by carbonic anhydrase or trypsin, preceding a heat treatment under vacuum.

2. Product according to claim 1, characterized in that it comes from cells of the mantle and mother of pearl of Pinctada Maxima, Margaritifera, or any other gasteropodous or cephalopodous bivalvular mollusc.

3. Product according to claim 1 or 2, characterized in that it is submitted to a mechanical treatment through crushing or ultrasounds.

4. Product according to anyone of the preceding claims, characterized in that it is submitted to an enzymatic deactivating chemical acid treatment.

5. Product according to anyone of the preceding claims, characterized in that it is submitted to an evaporating treatment at 50° during 48 hours.

6. Product according to anyone of the preceding claims, characterized in that it is filtered on a membrane, concentrated through centrifugation and lyophilized.

7. Product according to anyone of the preceding claims, characterized in that it is obtained by mixing the two phases in order to make a gel, a cream or a solution.

8. Product according to anyone of the preceding claims, characterized in that it can be mixed with excipients such as menthol, camphor, benjamin, citric acid, methyl oxybenzoate, stearylic alcohol, petrolatum, glycerol, purified water, vitamin A, alga extracts, calcium hydroxide, copra extracts or any other vehicle able to improve the effects thereof or to favour the penetration thereof.

9. Use of the product according to anyone of the preceding claims in the manufacture of a dermatologic composition.

10. Use of the product according to anyone of the preceding claims for the manufacture of a cosmetic product.
